# EUROPEAN PATENT SPECIFICATION

(11) **EP 4 013 483 B1**
(45) Date of publication and mention of the grant of the patent: **04.11.2026**
(21) Application number: 20742256.9
(22) Date of filing: 20.07.2020
(51) Int. Cl.: A61M 25/10, A61F 2/958

(54) **LEARNING ELECTRONIC BALLOON CATHETER SYSTEM FOR OPTIMAL STENT EMBEDDING**
LERNENDES ELEKTRONISCHES BALLONKATHETERSYSTEM ZUR OPTIMALEN STENTEINBETTUNG
SYSTÈME DE CATHÉTER À BALLONNET ÉLECTRONIQUE D'APPRENTISSAGE POUR UNE INTÉGRATION OPTIMALE DE STENT

(30) Priority: 14.08.2019 EP 19191676
(43) Date of publication of application: 22.06.2022
(73) Proprietor: BIOTRONIK AG, 8180 Bülach (CH)
(72) Inventor: WESSELMANN, Matthias, 8455 Rüdlingen (CH); WOLFER, Markus, 8451 Kleinandelfingen (CH); WERNLI, Jeremy, 5430 Wettingen (CH)
(74) Representative: WSL Patentanwälte Partnerschaft mbB
(86) International application number: PCT/EP2020/070417
(87) International publication number: WO 2021/028152

(56) References cited:
- US-A1- 2012 271 339
- US-A1- 2016 278 845
- US-A1- 2016 287 260
- US-A1- 2018 360 545

## Description

The present invention relates to a balloon catheter system for implanting a stent into a vessel, in particular for dilating a stenosis of the vessel.

Such a balloon catheter system comprises: a balloon extending in an axial direction, which surrounds a balloon interior of the balloon and has an outer surface facing away from the balloon interior; a stent crimped onto the outer surface of the balloon; and a catheter which is connected to the balloon, extends in the axial direction, and has a lumen via which the balloon interior may be filled with a fluid medium for expanding the balloon in a radial direction of the balloon, such that the stent may be dilated in the radial direction when the balloon expands.

When a stent is implanted at a narrowing, the stent must be dilated in such a way that a stent spring-back (what is known as 'recoil') and later vessel diameter fluctuations caused by pulse and blood pressure are already taken into account or compensated for.

In the prior art, it is suggested in this regard to perform a post-dilation with a high-pressure balloon as appropriate. Furthermore, methods are known with which an embedding of the stent in the vessel wall may be verified. In DE 10 2005 050 343 it is proposed in particular to carry out a final check using an image recording device. Furthermore, US 2012/0271339 proposes to measure the expansion of a balloon by means of an optical fibre.

In cases where the stent is not sufficiently over-expanded in the radial direction (i.e. towards the vessel wall) during dilation, the stent is often not in contact with the vessel wall everywhere from the beginning, and therefore the endothelium may not grow sufficiently over the stent. In addition, turbulences of the blood flow at individual struts of the stent may lead to local thromboses. In less pronounced cases of insufficient stent over-expansion, the stent may initially come into contact with the vessel wall, but there is a high probability that the vessel wall will be inflated and pushed away from the stent during the next phase of hypertension, in which case the freshly grown endothelial layer may be torn open again. This periodic injury may trigger a cascade of irritation, inflammation and even restenosis.

Furthermore, when placing stents in position, in particular drug-eluting stents, by means of appropriate catheter systems, there is regularly the risk that the stent is not sufficiently embedded if the treating physician does not know the recoil (in %) of the stent, which must be compensated for by overdilating the stent.

In addition, Compliance Data Charts regularly show only the maximum diameter of the catheter system during the procedure. Without precise measurement of the artery diameter, the diameter information in the Compliance Data Chart of the system used may be hardly any use at all for optimal overdilation.

Furthermore, X-ray images are only suitable to a limited extent for checking the embedding with regard to their achievable accuracy (+/- 10%). More precise measuring methods, such as intracoronary imaging procedures (for example intravascular ultrasound (IVUS) or optical coherence tomography (OCT)) are rarely used due to the high additional costs.

Furthermore, a high-pressure balloon is often used when implanting stents in order to improve the embedding in the vessel wall (so-called 'wall apposition'). High-pressure balloons are usually non-compliant balloons that may apply a high pressure to the vessel wall. However, this only happens as long as the balloon membrane is still folded. Once the balloon membrane has been expanded, for example because the vessel diameter has increased due to angioplasty, the balloon membrane absorbs the hydraulic pressure completely.

The high-pressure balloon, however, gives the treating physician the deceptive feeling that by further increasing the internal pressure of the balloon, the stent may be brought further into form even though the balloon diameter hardly changes, and thus hardly any more force may be transferred to the vessel or stent for deformation.

Non-compliant balloons are also usually only available in gradations of 0.25 mm or even 0.5 mm. Due to the risk of vessel dissection and the additional costs, the next-largest balloon is often not used, and for example the Dual Anti Platelet Therapy (DAPT) is relied upon.

IVUS/OCT offer the possibility to measure the vessel diameter after implantation. According to a study, OCT corresponds exactly to the diameter of the vessel, and IVUS overestimates the diameter of the vessel by about 3%. Both measuring means are costly in respect of time and material and are therefore not used systematically.

Both methods also have in common that they only measure the momentary wall contact or embedding for the recoiled stent under pulsatile load (usually with low blood pressure adjusted suitably for the intervention). They do not provide an answer as to whether the stent has sufficient elastic spring force to follow the vessel wall when blood pressure is elevated in order to thus prevent the endothelial layer from tearing open.

Inadequate embedding in patients for example with a tendency towards high blood pressure or in the case that sclerotic material (especially common in STEMI) is degraded/washed away may not be detected in this way.

US 2012/271339 A1 relates to an apparatus and method for determining an expanded diameter of a catheter balloon. An optical sensor includes an elongated optical fiber with at least one diffraction grating formed in a core of a distal portion thereof. The fiber distal portion is coupled to the inflatable balloon. Broadband light is transmitted to the diffraction grating by an optical interrogator. A portion of the light is reflected from the diffraction grating and is received by a wavelength detector. The wavelength of the reflected portion of light is dependent on the strain applied to the diffraction grating. The wavelength detector determines the strain in the balloon and correlates the strain to the diameter of the balloon.

US 2016/287260 A1 relates to systems and methods for operating a vessel occlusion catheter which may include a control and inflation device to control the filling of the balloon in such a manner that the vessel wall will not be overstressed while the safe occlusion of the blood vessel is achieved.

US 2018/360545 A1 relates to a system and method for tracking and determining characteristics of an inflatable medical instrument that is configured for interventional deployment. The system includes a guidewire that is positioned within a lumen of the inflatable medical instrument. The guidewire includes an optical fiber for a Fiber-Optical RealShape^{™} (FORS) system. The FORS system is configured to measure a shape of the guidewire during the interventional deployment of the inflatable medical instrument. A shape analysis module is configured to analyze the FORS data from the FORS system and determine characteristics of the inflatable medical instrument, including the diameter of the inflatable instrument, the pressurization of the instrument, whether the instrument has ruptured and the position of the inflatable instrument during an interventional procedure.

Based on this, the object of the present invention is therefore to provide a balloon catheter system of the type mentioned at the outset which allows an improved adaptation of the diameter of a stent to a vessel. In this way, better results may be achieved during angioplasty, and restenosis rates may be reduced. Furthermore, the risk of thrombosis and of dissection is reduced.

This object is achieved by a balloon catheter system having the features of claim 1.

The presently claimed invention provides a balloon catheter system according to claim 1.

Advantageous embodiments of the various aspects of the invention are specified in the corresponding dependent claims and are described below.

According to claim 1, a balloon catheter system, in particular for dilating a stenosis of a vessel of a patient, is disclosed, comprising:
a balloon extending in an axial direction, which surrounds a balloon interior of the balloon and has an outer surface facing away from the balloon interior;
a stent crimped onto the outer surface of the balloon; and
a catheter which is connected to the balloon, extends in the axial direction, and has a lumen via which the balloon interior may be filled with a fluid medium for expanding the balloon in a radial direction of the balloon, such that the stent may be dilated in the radial direction when the balloon expands.

In accordance with the invention it is provided that the balloon comprises at least one sensor, the at least one sensor being designed to repeatedly measure a current measurement value during the expansion of the balloon, which measurement value represents a measure of the expansion of the balloon in the radial direction of the balloon, the balloon catheter system further comprising a processing unit which is designed to terminate filling of the balloon interior with the medium and to prompt a display device to output a signal if the current measurement value or a current balloon parameter calculated therefrom reaches or exceeds a threshold value which is above a predefined reference value.

According to the invention, the processing unit is further configured to evaluate the following data: sensor data of the at least one sensor and case data, wherein the case data comprises a vessel diameter and a narrowing of the vessel. Further, the processing unit is configured to transmit the evaluated data to a database unit, wherein the database unit is configured to determine the threshold value on the basis of the data transmitted to the database unit.

The solution according to the invention preferably implements the finding that, when implanting a stent, the stent and the vessel, where possible, are over-expanded to such an extent that a dissection due to over-expansion during angioplasty remains unlikely, but at the same time the stent is sufficiently dilated that the stent and the vessel wall will no longer detach from one another.

The measurement value may be any measurand which - possibly in conjunction with one or more other variables - represents a measure of the expansion, in particular the diameter of the balloon, in the radial direction of the balloon, so that for example the current diameter of the balloon in the radial direction of the balloon may be determined or at least may be estimated on the basis of the current measurement value.

In the balloon catheter system according to the invention, it is provided that the reference value or reference diameter corresponds to a nominal vessel diameter of the vessel in which the stent is to be implanted. The nominal vessel diameter will be determined by means of zeroing within the scope of this application. The term "zeroing" means the diameter of the balloon when the cylindrical balloon diameter at its proximal and/or distal end corresponds to the diameter of the healthy vessel. This condition/diameter is also referred to as "0%" within the context of this application and forms the reference value or reference diameter. The reference diameter is determined during "zeroing" by means of X-ray control, a suitable imaging method and/or contact sensors at the distal and/or proximal end of the balloon.

Furthermore, in the balloon catheter system according to the invention it is provided that the threshold value exceeds the reference value or reference diameter by 5% to 20%, preferably 10%, in particular 8%. The percentage values for the diameter relate in each case to the reference diameter.

The necessary over-expansion of the nominal vessel diameter by the recommended 5% to 20%, preferably 10%, advantageously compensates for an elastic spring-back of the stent to smaller diameters (recoil), as well as for a squeezing of the vessel during a phase of hypertension that is likely to occur, and also keeps the risk of dissection relatively low.

According to the basic concept of the invention, the balloon catheter is designed in such a way that the reference diameter may be determined by means of zeroing and may then be exceeded in a controlled manner up to the threshold value. This ensures that the stent is sufficiently embedded in the vessel wall to prevent the stent and vessel wall from becoming detached from each other. At the same time, dissection of the vessel wall is avoided.

The sensor may, for example, measure the pressure in the balloon interior (for example at the proximal end of the balloon catheter) as a measurement value, and the current diameter of the balloon in the radial direction may be estimated from the current pressure, for example by a measurement curve or calibration showing the diameter as a function of the pressure in the balloon interior.

However, the sensor may also be designed, for example, as a strain sensor arranged on the balloon, by which the diameter may be measured in a more direct way. In this case, the measurement value thus may be transmitted for example as an output signal of the sensor to the processing unit, which then converts the measurement value in question into the corresponding current diameter of the balloon in the radial direction of the balloon. In this case the reference value represents a reference diameter of the balloon in the radial direction of the balloon.

The balloon catheter system may be designed to send the output signal of the strain sensor to the processing unit via a data line or telemetrically, in which case the at least one strain sensor is connected to a telemetry transmitter, which sends the output signal or corresponding data (for example by radio or ultrasound) to a telemetry receiver connected to the processing unit.

The telemetry transmitter may be implantable and may, for example, be arranged on a catheter shaft surrounding said lumen. Alternatively, the telemetry transmitter may be set up and intended to be arranged outside the patient. For example, the telemetry transmitter may be arranged on a catheter hub of the catheter so that the telemetry transmitter is arranged outside the patient.

The processing unit may also be designed to be implantable or may be set up and intended to be arranged outside the patient.

Furthermore, in accordance with an embodiment of the balloon catheter system according to the invention, it is provided that the balloon catheter system comprises an imaging unit for determining the reference value or reference diameter, and an input means for entering and/or confirming the reference diameter determined on the basis of an image (for example X-ray image) produced by the imaging unit.

Furthermore, according to an embodiment of the balloon catheter system according to the invention, it is provided that the balloon comprises contact sensors connected to the processing unit for determining the reference value or the reference diameter, which contact sensors are arranged in particular at the proximal and/or distal end of the cylindrical region of the balloon.

Furthermore, according to an embodiment of the balloon catheter system according to the invention, the balloon catheter system comprises a pump for filling the balloon interior with the fluid medium.

In accordance with a further embodiment of the balloon catheter system according to the invention, it is provided that the processing unit for automatically determining the reference value or the reference diameter is configured to prompt the pump to expand the balloon (in particular in steps) until all said contact sensors have contact with the vessel wall of a vessel into which the stent is implanted, the processing unit being configured to use the diameter of the balloon ultimately present with contact as the reference diameter.

Furthermore, according to an embodiment of the balloon catheter system of the invention, it is provided that the balloon catheter system is configured for user-controlled expansion of the balloon, the processing unit being configured to transmit the current diameter of the balloon to the display device, which is intended to display the current diameter of the balloon, and wherein the processing unit is configured to prompt the display device to output a signal (for example optical and/or acoustic) in case that the current diameter of the balloon reaches said threshold value. Furthermore, the processing unit may be configured to prompt the display unit to output a warning signal, in particular an optical and/or acoustic warning signal, if the current diameter of the balloon exceeds a maximum threshold value.

The maximum threshold value may correspond here to the reference diameter increased by 20%.

Furthermore, according to an alternative embodiment of the balloon catheter system according to the invention, it is provided that the processing unit is configured to control the pump for expanding the balloon so that the current diameter of the balloon automatically strives to reach the threshold value. Here too, the processing unit may be configured to transmit the current diameter of the balloon to the display device, which is intended to display the current diameter of the balloon.

Furthermore, according to an embodiment of the balloon catheter system according to the invention, it is provided that the processing unit is set up and intended to record sensor data of the at least one sensor (for example strain sensor), case data, acute result data of the stent implantation or angioplasty, as well as clinical result data of the stent implantation or angioplasty.

The sensor data may be constituted by one or more of the following items of information: a diameter of the balloon, a pressure inside the balloon when zeroing, a maximum relative expansion of the balloon [for example in %], or a maximum pressure in the balloon interior.

The case data may be constituted by one or more of the following items of information: patient data, such as age and gender; a vessel diameter of the vessel to be dilated (for example measured by an imaging technique, such as X-ray), a narrowing of the vessel [for example in %], a length of the stenosis of the vessel, overlapping implants/stents already implanted.

The acute result data may be constituted by one or more of the following items of information: reference diameter/balloon diameter at "0%" (in mm) , hydraulic internal balloon pressure at "0%" (in atm)/reference diameter, maximum balloon diameter (in mm), maximum relative radial expansion beyond the healthy vessel diameter (in %), presence of a perforation/dissection [y/n],

The clinical result data ("follow-up") may include, for example, the following information: time from intervention to restenosis at the same site in the vessel, vascular rupture (also known as target lesion failure (TLF)), revascularisation of downstream tissue (also known as target lesion revascularisation (TLR)).

The processing device or a further computer unit is preferably used or preferably configured to suitably evaluate the aforementioned data (or a selection of the aforementioned data), in particular to correlate them, so that an optimum threshold value may be specified for a specific type of patient. These threshold values are preferably stored in a database of a database unit and are preferably updated continuously.

Furthermore, according to an embodiment of the balloon catheter system according to the invention, it is provided that the processing unit is configured to retrieve the threshold value from said database, in particular via a remote data transmission connection (for example via the Internet).

A further aspect of the present disclosure relates to a method for expanding a balloon of a balloon catheter system, in particular a balloon catheter system according to the invention, wherein, by means of at least one sensor, during the expansion of the balloon in the radial direction of the balloon a current measurement value is measured, which measurement value represents a measure for the expansion of the balloon in the radial direction of the balloon, the expansion of the balloon being terminated and/or a signal being displayed if the current measurement value or a current balloon parameter calculated therefrom reaches or exceeds a threshold value which is above a predefined reference value.

It is provided here that the current balloon parameter is a current diameter of the balloon in the radial direction of the balloon, the reference value being a reference diameter of the balloon in the radial direction of the balloon and corresponding to the nominal vessel diameter of a vessel in which the stent is implanted, the threshold value exceeding the reference diameter by 5% to 20%, preferably 10%. Here, the nominal vessel diameter is the diameter of the balloon when the balloon contacts the vessel wall, as already described above.

Furthermore, according to an embodiment of the method it is provided that an imaging method is used to determine the reference value or the reference diameter, or that the reference diameter is determined automatically by expanding the balloon until it has a circumferential contact with the vessel wall of a vessel into which the stent is implanted, the diameter of the balloon at the time of the contact being used as the reference diameter.

Furthermore, according to an embodiment of the method it is provided that the balloon is expanded continuously or in steps by a user, said current diameter of the balloon being continuously determined and is indicated (for example optically and/or acoustically), wherein in particular a warning signal (for example optical and/or acoustic) is provided if the current diameter of the balloon exceeds a maximum threshold value (see above).

According to an alternative version of the method, it is provided that the expansion of the balloon is controlled so that the current diameter of the balloon automatically reaches the threshold value. Here too, the current diameter in particular is displayed to the user continuously.

Furthermore, according to an embodiment of the method it is provided that the following data are detected by means of the processing unit: sensor data of the at least one sensor and/or case data and/or acute result data and/or clinical result data (see above), wherein the processing unit is further preferably configured to transmit the detected data to a database unit of the balloon catheter system, and wherein the database unit automatically determines the threshold value on the basis of the data transmitted to the database unit and proposes it to the user or physician.

The threshold value used may thus be generated and made available by the database unit (in particular via a remote data transmission connection) in a manner suitable for a patient, wherein in particular the threshold values stored in a database of the database unit are continuously updated, in particular in that sensor data of the at least one strain sensor, case data, acute result data and clinical result data are continuously recorded and used to determine current threshold values, for example by suitable automatic correlation of the data. Hereinafter, features, advantages and embodiments of the present invention shall be explained with reference to the drawing, in which:

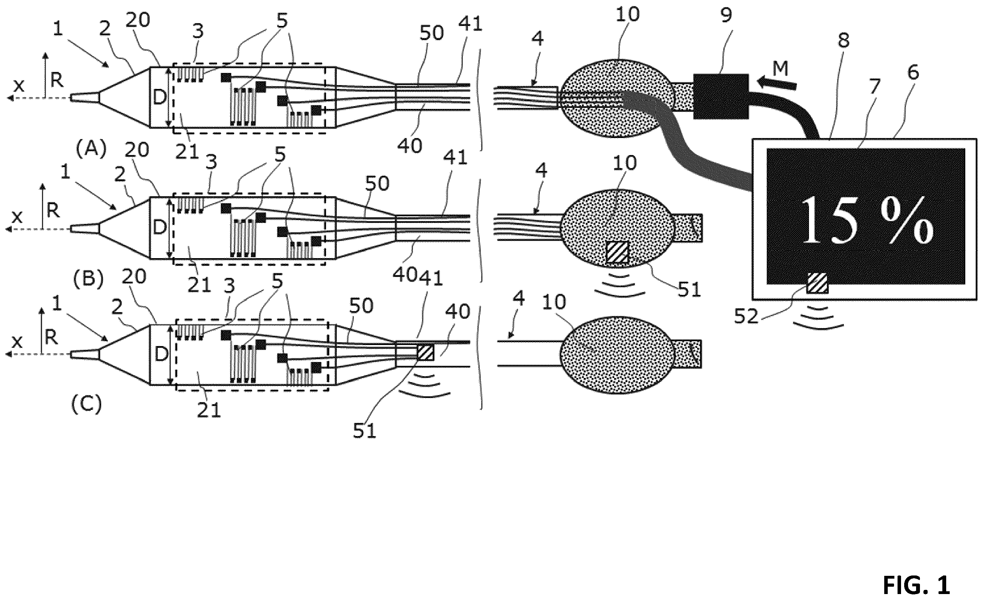

- Fig. 1: shows different embodiments of a balloon catheter system according to the invention, wherein the at least one strain sensor is connected to the processing unit via a data line (A), or wherein the strain sensor is connected to the processing unit via telemetry (B) or (C).

Figure 1 shows different embodiments of a balloon catheter system 1 according to the invention. The individual configurations of the balloon catheter system 1 each have a balloon 2 extending in an axial direction x, which surrounds a balloon interior 21 of the balloon 2 and has an outer surface 20 facing away from the balloon interior 21, as well as a stent 3 (in particular metallic) crimped onto the outer surface 20 of the balloon 2. Furthermore, a catheter 4 is provided in each case, which is connected to the balloon 2 and extends in the axial direction x and has a lumen 40, via which the balloon interior 21 may be filled with a fluid medium M for expanding the balloon 2 in a radial direction R of the balloon 2, so that the stent 3 may be dilated in the radial direction R when the balloon 2 expands. Here, according to an embodiment of the invention, it is provided that the balloon 2 has at least one strain sensor 5, here for example in each case three strain sensors 5, wherein the at least one strain sensor 5 is designed to provide an output signal, wherein the balloon catheter system 1 further has a processing unit 6, which is designed to calculate a current diameter D of the balloon 2 in the radial direction R during expansion of the balloon 2 on the basis of the output signal, wherein the processing unit 6 is designed to terminate filling of the balloon interior 21 with the medium M and/or to prompt a display device 7 to output a signal when the current diameter D reaches or exceeds a threshold value which is above a predefined reference diameter.

As shown in Fig. 1(A), the at least one or more strain sensors 5, which are for example located on the outer surface 20 of the balloon 2, may be connected to the processing unit 6 via two conductors 50 each.

Alternatively, the measured data may be sent to a telemetry receiver 52 connected to the processing unit 6 by means of a telemetry transmitter 51. The telemetry transmitter 51 may be designed to be implantable and may, for example, be arranged on a catheter shaft 41 of catheter 4 surrounding the lumen 40, adjacently to the balloon 2 (see Fig. 1(C)). Furthermore, the telemetry transmitter 51 may also be located on a catheter hub 10, for example.

In all embodiments, said medium M is preferably pressed into the balloon interior 21 by means of a pump 8, which is connected for example via a Luer connection 9 to the catheter 4 or the lumen 40, in order to unfold or expand the balloon 2 in the radial direction.

For the over-expansion measurement on the balloon 2, it is advantageous if the balloon 2 is no longer folded at the nominal vessel diameter. The balloon diameter is therefore in particular smaller than the nominal vessel diameter of the vessel in which the stent 3 is to be implanted.

To obtain a reference value for the required over-expansion of the balloon 2, the expansion of balloon 2 is first measured at the nominal vessel diameter. There are several possibilities for detecting the nominal vessel diameter.

For example, according to one embodiment, the treating physician may place the stent during an accompanying imaging (for example X-ray imaging) and dilate it in the radial direction R until the current diameter D of the balloon and adjacent healthy vessel connections form a continuous line. In this case, the treating physician or user of the balloon catheter system 1 for example may confirm the current diameter as a reference diameter for the system 1 (for example by pressing a button).

Alternatively, the treating physician may place the stent 3 in the stenosis and give a signal to system 1 to determine the nominal vessel diameter. The system 1 increases the pressure in the balloon interior 21 appropriately in steps by means of the pump 8 until contact sensors, which are distributed on a cylindrical part of the balloon 2 over the circumference of the balloon 2, are all in contact with the vessel wall at both balloon ends. It is advantageous if the balloon 2 protrudes from the stenosis on at least one side.

The relative expansion of the balloon 2 is now increased by increasing the pressure in the balloon interior 21 (balloon internal pressure) until the over-expansion is in the target range of from 5% to 20%, preferably 10% over-expansion.

There are a number of ways to adjust the optimal over-expansion of the stent and the vessel.

According to one embodiment, it is provided that the treating physician increases the internal pressure of the balloon in steps, while the system 1 continuously displays the over-expansion of the stent 3 by means of the display device 7. The system 1 signals that the optimal expansion range (threshold value) has been reached and warns when the optimal over-expansion has been exceeded or when a maximum threshold value has been exceeded. The device increases the internal pressure of the balloon in steps in a closed control loop until the desired over-expansion is measured on the balloon.

The case in question is preferably classified by the treating physician (for example patient, age, branch, type of stenosis, condition), the procedure described (nominal diameter, over-expansion diameter, set stent diameter, dissection y/n, etc.).

In the case of a restenosis at the site of the stent, for example the duration and severity of the stenosis is reported back by the treating physician.

The data are preferably evaluated periodically for correlations, and the recommended action of over-expansion (threshold value) is revised by experts and/or an algorithm if necessary.

The processing unit 6 continuously receives, preferably via an Internet connection, the current recommended actions for optimal implantation of the stent 3.

Preferably, the balloon 2 is equipped with at least one strain sensor 5 in the cylindrical region of the balloon 2. Both components are preferably designed for a compliance of up to at least 5%, ideally above 30%, and a resolution of +/-5%, preferably +/-1%, without delamination or risking of a line break.

The at least one strain sensor 5 is supplied with energy via a power line, in particular in accordance with one embodiment. The energy may come from a power source outside the body, but may also be provided by a suitable battery or antenna inside the body.

The processing unit 6, or an algorithm implemented on it, preferably continuously calculates the over-expansion of the balloon 2 beyond the diameter of the healthy vessel portion adjacent to the stenosis on the basis of the measured data of the expansion sensor 5 and constantly checks whether the target range of the over-expansion or of said threshold value has been reached, and announces this acoustically or visually. If the recommended over-expansion range (maximum threshold value) is exceeded, there is preferably a further signal, which through its frequency for example communicates the increased risk of rupture to the user.

Alternatively, the control unit may also take over the implantation fully automatically via a control loop consisting of over-expansion measurement (target value) and pressure setting via a (for example digital) pump 8.

The data of the procedure are preferably recorded, together with the classification of the stenosis, by the processing unit 6 and the procedural success is in particular documented and preferably transmitted to a database of a central database unit. The database unit may be formed by one or more suitably networked computers.

These data are particularly suitable for determining a correlation between the over-expansion range or threshold value and clinical success and for returning improved recommended actions to the algorithm.

Furthermore, in accordance with one embodiment the balloon 2 may be manufactured for example from all thermoplastic balloon materials which offer a sufficiently high pressure resistance with low wall thickness. These are preferably polymers from the polyamide family or thermoplastic polyurethanes. The balloon-forming process is preferably designed to produce a balloon behaviour that is distinctly semi-compliant and offers sufficient flexibility for the diameter range in the application.

The expansion D of the balloon in the circumferential direction (preferably the diameter D in the cylindrical region of the balloon 2) may be determined in many different ways.

For example, the at least one strain sensor 5 may be designed as a strain gauge in which the change in electrical resistance of a conductor track is used as a measure of its expansion, which in turn is proportional to the change in circumference or diameter of the balloon.

Alternatively, a measurement of the change in the resistance of the fluid column in the balloon may be performed. In this case, the medium M must be electrically conductive.

Furthermore, the at least one strain sensor 5 may be a capacitive strain sensor 5, which determines the change of the balloon membrane when it becomes thinner or larger due to the expansion.

Furthermore, the strain state of the balloon 2 may be determined by means of an ultrasonic measurement.

Lastly, an additionally applied pressure or an additionally inflated liquid volume may be used to draw conclusions about the expansion state of the balloon.

Furthermore, according to one embodiment, the lines 50 and the at least one strain sensor 5 (in the case of a strain gauge) must have sufficient expansibility and must be significantly softer than the balloon membrane in order to change the balloon compliance as little as possible and to prevent delamination of the strain sensor from the balloon 2. For this purpose, the conductors 50 or the at least one strain sensor may have a meandering course (especially in the case of non-elastic or slightly elastic conductor tracks). Furthermore, the conductors may be made for example of conductive, expandable inks/pastes based on metal nanoparticles, or expandable conductive polymers such as PEDOT:PSS.

As already indicated above, the processing unit 6 is preferably set up and intended for recording sensor data of the at least one strain sensor 5, case data, acute result data, as well as clinical result data.

The sensor data are preferably for example a temporal course of the balloon diameter and of the simultaneously existing hydraulic pressure in the balloon interior, the exact time, diameter and pressure when the original vessel diameter is restored, a maximum relative expansion of the balloon [for example in %] in relation to the original vessel diameter (reference diameter), and the maximum pressure in the balloon interior during the procedure.

The case data are, for example: patient data (for example age and sex), a vessel diameter of the vessel to be dilated (for example measured using an imaging procedure, for example, X-ray), a narrowing of the vessel [for example, in %], a length of the stenosis of the vessel, and whether and how far the stent has been placed overlapping an existing stent.

The acute result data are, for example:
- a description of the complication, such as perforation, dissection,
- a dilation diameter of the stent (in mm), from which the over-expansion in relation to the original vessel diameter is calculated.

The clinical result data may include, for example, the following information: time from surgery to restenosis at the same site in the vessel.

The present invention allows advantageously an optimisation of the so-called 'wall apposition' by optimal over-expansion of the stent 3 and vessel.

This results in a better endothelialisation of the stent 3, since a suitably pretensioned stent 3 will no longer lose contact with the vessel wall, not even in the case of high blood pressure.

Furthermore, the fear of an imminent dissection is removed for the treating physician by the warning aid in the event of over-expansion.

Furthermore, the system 1 according to the invention has the advantage of being adaptive and, in particular, allows procedural and clinical success to be improved by learning the optimal stent over-expansion.

Due to the invention, reading errors regarding the Compliance Data Chart may be avoided.

Lastly, balloon dilation as an indication of the efficacy of the procedure has proven to be more appropriate than balloon internal pressure, which may give an erroneous feeling of effectiveness (see above).

## Claims

1. A balloon catheter system (1) comprising:
a balloon (2) extending in an axial direction (x), which surrounds a balloon interior (21) of the balloon (2) and has an outer surface (20) facing away from the balloon interior (21);
a stent (3) crimped onto the outer surface (20) of the balloon;
a catheter (4) which is connected to the balloon (2), extends in the axial direction (x), and has a lumen (40) via which the balloon interior (21) may be filled with a fluid medium (M) for expanding the balloon (2) in a radial direction (R) of the balloon (2), such that the stent (3) may be dilated in the radial direction (R) when the balloon (2) expands;
and a database unit;
wherein the balloon catheter system (2) comprises at least one sensor (5), the at least one sensor (5) being designed to measure a current measurement value during the expansion of the balloon (2), which measurement value represents a measure of the expansion of the balloon (2) in the radial direction (R) of the balloon (2), the balloon catheter system (1) further comprising a processing unit (6) which is designed to terminate filling of the balloon interior (21) with the medium (M) and to prompt a display device (7) to output a signal if the current measurement value or a current balloon parameter (D) determined therefrom reaches a threshold value which is above a predefined reference value, the current balloon parameter being a current diameter (D) of the balloon (2) in the radial direction (R) of the balloon (2), the reference value corresponding to the balloon diameter upon contact of the proximal and/or distal balloon end with a vessel wall, wherein the processing unit (6) is configured to evaluate the following data: sensor data of the at least one sensor (5) and case data, wherein the case data comprises a vessel diameter and a narrowing of a vessel, the processing unit (6) is further configured to transmit the evaluated data to the database unit, wherein the database unit is configured to determine the threshold value on the basis of the data transmitted to the database unit.

2. The balloon catheter system according to claim 1, **characterised in that** the sensor (5) is a strain sensor (5) which is designed to provide the current measurement value in the form of an output signal, the processing unit (6) being designed to calculate the current diameter (D) of the balloon (2) on the basis of the output signal, and the reference value being a reference diameter of the balloon (2) in the radial direction (R) of the balloon (2).

3. The balloon catheter system according to any one of the preceding claims, **characterised in that** the balloon catheter system (1) comprises an imaging unit for determining the reference value, and an input means for entering and/or confirming the reference value.

4. The balloon catheter system according to either one of claims 1 to 2, **characterised in that** the balloon (2) comprises contact sensors for determining the reference value.

5. The balloon catheter system according to any one of the preceding claims, **characterised in that** the balloon catheter system (1) comprises a pump (8) for filling the balloon interior (21) with the fluid medium (M).

6. The balloon catheter system according to claims 4 and 5, **characterised in that** the processing unit (6) for determining the reference value is configured to prompt the pump (8) to expand the balloon (2) until all contact sensors have signalled contact with the vessel wall of a vessel into which the stent (3) is implanted, the processing unit (5) being configured to use the diameter (D) of the balloon (2) then present as the reference value.

7. The balloon catheter system according to any one of the preceding claims, **characterised in that** the balloon catheter system (1) is configured for user-controlled expansion of the balloon (2), the processing unit (6) being configured to transmit the current value of the balloon parameter (D) to the display device (7), which is intended to display the current value of the balloon parameter (D).

8. The balloon catheter system according to any one of claims 1 to 6, **characterised in that** the processing unit (6) is configured to control the pump (8) for expanding the balloon (2) so that the current value of the balloon parameter (D) strives to reach the threshold value.

## Patentansprüche

1. Ballonkathetersystem (1), umfassend:
einen Ballon (2), der sich in eine axialen Richtung (x) erstreckt, der ein Balloninneres (21) des Ballons (2) umgibt und eine Außenfläche (20) aufweist, die von dem Balloninneren (21) abgewandt ist;
einen Stent (3), der auf die Außenfläche (20) des Ballons gekrimpt ist;
einen Katheter (4), der mit dem Ballon (2) verbunden ist, sich in die axiale Richtung (x) erstreckt und ein Lumen (40) aufweist, über welches das Balloninnere (21) mit einem fluiden Medium (M) zum Expandieren des Ballons (2) in einer radialen Richtung (R) des Ballons (2) derart gefüllt werden kann, dass der Stent (3) in der radialen Richtung (R) dilatiert werden kann, wenn der Ballon (2) expandiert;
und eine Datenbankeinheit;
wobei das Ballonkathetersystem (2) mindestens einen Sensor (5) umfasst, wobei der mindestens eine Sensor (5) dazu ausgebildet ist, während der Expansion des Ballons (2) einen aktuellen Messwert zu messen, wobei der Messwert ein Maß der Expansion des Ballons (2) in die radiale Richtung (R) des Ballons (2) darstellt, wobei das Ballonkathetersystem (1) ferner eine Verarbeitungseinheit (6) umfasst, die dazu ausgebildet ist, das Füllen des Balloninneren (21) mit dem Medium (M) zu beenden und
eine Anzeigevorrichtung (7) zu veranlassen, ein Signal auszugeben, wenn der aktuelle Messwert oder ein daraus bestimmter aktueller Ballonparameter (D) einen Schwellenwert erreicht, der oberhalb eines vordefinierten Referenzwertes liegt, wobei der aktuelle Ballonparameter ein aktueller Durchmesser (D) des Ballons (2) in der radialen Richtung (R) des Ballons (2) ist, wobei der Referenzwert dem Ballondurchmesser bei Kontakt des proximalen und/oder distalen Ballonendes mit einer Gefäßwand entspricht, wobei die Verarbeitungseinheit (6) dafür konfiguriert ist, die folgenden Daten auszuwerten: Sensordaten des mindestens einen Sensors (5) und Falldaten, wobei die Falldaten einen Gefäßdurchmesser und eine Verengung eines Gefäßes umfassen, wobei die Verarbeitungseinheit (6) ferner derart konfiguriert ist, die ausgewerteten Daten an die Datenbankeinheit zu übertragen, wobei die Datenbankeinheit dafür konfiguriert ist, den Schwellenwert auf der Grundlage der an die Datenbankeinheit übertragenen Daten zu bestimmen.

2. Ballonkathetersystem nach Anspruch 1, **dadurch gekennzeichnet, dass** der Sensor (5) ein Dehnungssensor (5) ist, der dazu ausgebildet ist, den aktuellen Messwert in Form eines Ausgangssignals bereitzustellen, wobei die Verarbeitungseinheit (6) dazu ausgebildet ist, den aktuellen Durchmesser (D) des Ballons (2) auf der Grundlage des Ausgangssignals zu berechnen, und wobei der Referenzwert ein Referenzdurchmesser des Ballons (2) in der radialen Richtung (R) des Ballons (2) ist.

3. Ballonkathetersystem nach einem der vorhergehenden Ansprüche, **dadurch gekennzeichnet, dass** das Ballonkathetersystem (1) eine Bildgebungseinheit zum Bestimmen des Referenzwertes und ein Eingabemittel zum Eingeben und/oder Bestätigen des Referenzwertes umfasst.

4. Ballonkathetersystem nach einem der Ansprüche 1 bis 2, **dadurch gekennzeichnet, dass** der Ballon (2) Kontaktsensoren zum Bestimmen des Referenzwertes umfasst.

5. Ballonkathetersystem nach einem der vorhergehenden Ansprüche, **dadurch gekennzeichnet, dass** das Ballonkathetersystem (1) eine Pumpe (8) zum Füllen des Balloninneren (21) mit dem fluiden Medium (M) umfasst.

6. Ballonkathetersystem nach den Ansprüchen 4 und 5, **dadurch gekennzeichnet, dass** die Verarbeitungseinheit (6) zum Bestimmen des Referenzwertes konfiguriert ist, die Pumpe (8) zu veranlassen, den Ballon (2) zu expandieren, bis alle Kontaktsensoren Kontakt mit der Gefäßwand eines Gefäßes signalisiert haben, in das der Stent (3) implantiert wird, wobei die Verarbeitungseinheit (5) dazu konfiguriert ist, den dann eingenommenen Durchmesser (D) des Ballons (2) als den Referenzwert zu verwenden.

7. Ballonkathetersystem nach einem der vorhergehenden Ansprüche, **dadurch gekennzeichnet, dass** das Ballonkathetersystem (1) zur benutzergesteuerten Expansion des Ballons (2) konfiguriert ist, wobei die Verarbeitungseinheit (6) dazu konfiguriert ist, den aktuellen Wert des Ballonparameters (D) an die Anzeigevorrichtung (7) zu übertragen, die dazu bestimmt ist, den aktuellen Wert des Ballonparameters (D) anzuzeigen.

8. Ballonkathetersystem nach einem der Ansprüche 1 bis 6, **dadurch gekennzeichnet, dass** die Verarbeitungseinheit (6) dazu konfiguriert ist, die Pumpe (8) zum Expandieren des Ballons (2) so zu steuern, dass der aktuelle Wert des Ballonparameters (D) danach strebt, den Schwellenwert zu erreichen.

## Revendications

1. Système de cathéter à ballonnet (1) comprenant : un ballonnet (2) s'étendant dans une direction axiale (x), qui entoure un intérieur de ballonnet (21) du ballonnet (2) et présente une surface extérieure (20) tournée à l'opposé de l'intérieur de ballonnet (21); un stent (3) serti sur la surface extérieure (20) du ballonnet ; un cathéter (4) qui est relié au ballonnet (2), s'étend dans la direction axiale (x) et présente une lumière (40) par laquelle l'intérieur de ballonnet (21) peut être rempli d'un milieu fluide (M) pour dilater le ballonnet (2) dans une direction radiale (R) du ballonnet (2), de telle sorte que le stent (3) peut être dilaté dans la direction radiale (R) lorsque le ballonnet (2) se dilate ; et une unité de base de données ; le système de cathéter à ballonnet (2) comprenant au moins un capteur (5), l'au moins un capteur (5) étant conçu pour mesurer une valeur de mesure actuelle pendant la dilatation du ballonnet (2), laquelle valeur de mesure représente une mesure de la dilatation du ballonnet (2) dans la direction radiale (R) du ballonnet (2), le système de cathéter à ballonnet (1) comprenant en outre une unité de traitement (6) qui est conçue pour mettre fin au remplissage de l'intérieur de ballonnet (21) avec le milieu (M) et pour amener un dispositif d'affichage (7) à émettre un signal si la valeur de mesure actuelle ou un paramètre de ballonnet actuel (D) déterminé à partir de celle-ci atteint une valeur seuil qui est supérieure à une valeur de référence prédéfinie, le paramètre de ballonnet actuel étant un diamètre actuel (D) du ballonnet (2) dans la direction radiale (R) du ballonnet (2), la valeur de référence correspondant au diamètre du ballonnet lors du contact de l'extrémité proximale et/ou distale du ballonnet avec une paroi de vaisseau, l'unité de traitement (6) étant configurée pour évaluer les données suivantes : des données de capteur de l'au moins un capteur (5) et des données de cas, les données de cas comprenant un diamètre de vaisseau et un rétrécissement d'un vaisseau, l'unité de traitement (6) étant en outre configurée pour transmettre les données évaluées à l'unité de base de données, l'unité de base de données étant configurée pour déterminer la valeur seuil sur la base des données transmises à l'unité de base de données.

2. Système de cathéter à ballonnet selon la revendication 1, **caractérisé en ce que** le capteur (5) est un capteur de déformation (5) qui est conçu pour fournir la valeur de mesure actuelle sous la forme d'un signal de sortie, l'unité de traitement (6) étant conçue pour calculer le diamètre actuel (D) du ballonnet (2) sur la base du signal de sortie, et la valeur de référence étant un diamètre de référence du ballonnet (2) dans la direction radiale (R) du ballonnet (2).

3. Système de cathéter à ballonnet selon l'une quelconque des revendications précédentes, **caractérisé en ce que** le système de cathéter à ballonnet (1) comprend une unité d'imagerie pour déterminer la valeur de référence, et un moyen d'entrée pour saisir et/ou confirmer la valeur de référence.

4. Système de cathéter à ballonnet selon l'une ou l'autre des revendications 1 à 2, **caractérisé en ce que** le ballonnet (2) comprend des capteurs de contact pour déterminer la valeur de référence.

5. Système de cathéter à ballonnet selon l'une quelconque des revendications précédentes, **caractérisé en ce que** le système de cathéter à ballonnet (1) comprend une pompe (8) pour remplir l'intérieur de ballonnet (21) avec le milieu fluide (M).

6. Système de cathéter à ballonnet selon les revendications 4 et 5, **caractérisé en ce que** l'unité de traitement (6) destinée à déterminer la valeur de référence est configurée pour amener la pompe (8) à dilater le ballonnet (2) jusqu'à ce que tous les capteurs de contact aient signalé un contact avec la paroi de vaisseau d'un vaisseau dans lequel le stent (3) est implanté, l'unité de traitement (5) étant configurée pour utiliser le diamètre (D) du ballonnet (2) alors présent comme valeur de référence.

7. Système de cathéter à ballonnet selon l'une quelconque des revendications précédentes, **caractérisé en ce que** le système de cathéter à ballonnet (1) est configuré pour une dilatation du ballonnet (2) commandée par l'utilisateur, l'unité de traitement (6) étant configurée pour transmettre la valeur actuelle du paramètre de ballonnet (D) au dispositif d'affichage (7), qui est destiné à afficher la valeur actuelle du paramètre de ballonnet (D).

8. Système de cathéter à ballonnet selon l'une quelconque des revendications 1 à 6, **caractérisé en ce que** l'unité de traitement (6) est configurée pour commander la pompe (8) en vue de dilater le ballonnet (2) de sorte que la valeur actuelle du paramètre de ballonnet (D) tende à atteindre la valeur seuil
